# EUROPEAN PATENT APPLICATION

(11) **EP 4 624 563 A1**
(43) Date of publication of application: **01.10.2025**
(21) Application number: 24165955.6
(22) Date of filing: 25.03.2024
(51) Int. Cl.: C12M 3/00, C12M 1/36, G01N 1/20

(54) **METHOD FOR PERFORMING A BIOPROCESS ON IMMUNE OR NAIVE CELL CULTURES**

(71) Applicant: The Automation Partnership (Cambridge) Ltd., Hertfordshire SG8 5WY (GB)
(72) Inventor: Stacey, Adrian, Royston, SG8 5WY (GB); Binninger, Steven, Royston, SG8 5WY (GB)
(74) Representative: Cohausz & Florack

(57) **Abstract**

The invention relates to a method for performing a bioprocess on immune or naive cell cultures to obtain processed cell cultures on a semi-automated bioprocessing system (1), comprising a primary workstation (2) and performing a workflow, the workflow comprising a plurality of unit operations performed by the primary workstation (2), wherein the primary workstation (2) comprises a unit operation interface (4), wherein the unit operations are performed by an interaction of the unit operation interface (4) with at least one cartridge (5), wherein a first unit operation (16) and a second unit operation (17) of the plurality of unit operations are performed by the unit operation interface (4) with different cartridge configurations (13), wherein between the first and the second unit operation (16, 17) the cartridge configuration (13) is manually reconfigured in a reconfiguration step, wherein the manual reconfiguration in the reconfiguration step is supported by the bioprocessing system (1).

## Description

The present invention relates to a method for performing a bioprocess on immune or naive cell cultures according to claim 1, to a bioprocessing system for performing a bioprocess on immune or naive cell cultures according to claim 13 and to a method for performing a further iteration of the bioprocess performed according to the proposed method according to claim 14.

In recent years, the field of cell and gene therapy has garnered increasing attention. As promising therapies multiply, the focus intensifies on scaling up production systems. Some biotech firms are actively pursuing fully automated systems to simultaneously produce several batches of immune or naive cell cultures for cell and gene therapy. However, these systems encounter unique challenges. For example, cell and gene therapy may not have reached a stage where production capacities are the primary bottleneck, and there might not be enough experience for a fully automated system to enter the market at profitable rates. This renders the development of such a system both challenging and cost-intensive. As customers are also still inexperienced with automated systems and extremely focused on reliability over speed, gaining customer approval entails mastering a number of complex challenges simultaneously.

One of the main reason integrated systems are not widely used is that wholesale transfer of a process onto an integrated system requires first that the component parts of that process be verified on the integrated system. For many proposed systems there is no route by which individual component parts (unit operations) can be tested in isolation, without investment in the full system, and without obscuring the behavior of those individual components because they can only be run in conjunction with other components in the system (e.g., setting up a complex cartridge / tube-set), or because material moving into them and out of them cannot be objectively assessed offline (on separate equipment) as there is no way of getting an entirely representative sample out or ensuring the nature of the input material. A verification of the system is therefore very difficult.

The invention is based on the problem of providing a method for performing a bioprocess and a corresponding bioprocessing system that improve the current manual methods and at the same time are less challenging to build and accept.

The above-noted object is solved by the features of claim 1.

The main realization of the present invention is that a good balance between manual and automatic actions allows designing around many challenges in automation, e.g. complex transport processes. It is further possible to gradually move from a manual process towards a fully automated process by providing clear boundaries between automation and manual work which can be moved by adding unit operations one by one and at some point also automating the connection between the unit operations. With the proposed system, the manual processes relate specifically to movement of material between unit operations, and therefore total flexibility is retained in terms of running a short or long process (a single unit operation, just a few unit operations, or many unit operations), in routing material to external components and back (if the relevant unit operations are not covered by the system), and in system configuration.

Another crucial realization is that the proposed system provides a small number of physical units like a cartridge that can be handled by a system and by a human. The system therefore allows evaluating the automation of single unit operations and moving towards a fully automated system step by step.

Further, the system allows automating tasks that have a higher chance of failing if performed by a human. The right combination of automation and user support leads to a system which is less prone to errors than manual systems and automatic systems, as the user performs tasks that are hard to automate with very low error rates supported by the system and the system performs tasks that are error-prone when performed manually. Additionally, the system may perform tasks that are time-intensive in a semi-automated manner.

In summary, it is proposed to use one system interface for multiple unit operations and having the user reconfigure a cartridge configuration used in the unit operations between the unit operations supported by the system. Such a system provides a high modularity by using cartridges instead of specialized system components for different unit operations and is easy to operate as the system interface doesn't change and does not need to automate highly complex reconfigurations of the cartridge configuration as the user does these. At the same time, the system guides and preferably controls the user actions. This approach results in a system that combines the benefits of both manual and automated processes, reducing overall errors and improving overall process efficiency.

In detail, a method for performing a bioprocess on immune or naive cell cultures to obtain processed cell cultures on a semi-automated bioprocessing system, wherein the bioprocessing system comprises a primary workstation, wherein for performing the bioprocess the bioprocessing system performs a workflow, wherein the workflow comprises a plurality of unit operations performed by the primary workstation, wherein the primary workstation comprises a unit operation interface, wherein the unit operations are performed by an interaction of the unit operation interface with at least one cartridge, wherein the cartridge, optionally together with containers, placed at, in particular on, the unit operation interface define a cartridge configuration, wherein the unit operation interface comprises at least one active element interacting with the at least one cartridge to perform at least one unit operation of the plurality of unit operations, wherein a first unit operation and a second unit operation of the plurality of unit operations are performed by the unit operation interface with different cartridge configurations, wherein between the first and the second unit operation the cartridge configuration is manually reconfigured in a reconfiguration step, wherein the manual reconfiguration in the reconfiguration step is supported by the bioprocessing system is proposed.

The active element may transfer mechanical energy and/or may be used for two unit operations with different cartridges (claim 2). For example, the active element may be a rotating shaft driving a functional element inside the cartridge like a centrifuge. Having a standardized interface for a user to place cartridges on, for example, makes (re)configuring the cartridge configuration at least to some extent independent of the unit operation, thereby reducing the chance for errors. The ability to perform various unit operations with the same or different cartridges and containers allows the system to handle a wide range of cell and gene therapy applications.

One target for automation are fluidic connections (claim 3). These may be present between the cartridge and other containers on the unit operation interface and the unit operation interface may actively transfer fluids between a container and the cartridge. Combining multiple unit operations using the same active element and fluidically connecting containers to cartridges results in faster processing times by minimizing the need for manual intervention and material transfer steps.

Embodiments according to claim 4 relate to making fluidic connections with a tube welder. Connecting and disconnecting tubes is a risk for contamination if performed manually and takes a lot of time. Without automatic tube welding, each time a connection needs to be made, the system has to wait for a user. By automizing the (sterile) connections, the system may perform many consecutive operations without user intervention. The possibility of mistakes is reduced.

According to claim 5, welding may be done at the unit operation interface directly or by transporting the cartridge and/or container to a welding interface. If the interfaces are located close to each other, this short transport may be less complicated than automating all transports for the system, as, for example, conveyors can be used.

It is possible to use one cartridge for more than one unit operation after reconfiguration of the cartridge configuration, for example by exchanging containers connected to the cartridge (claim 6). This embodiment shows how a guided manual reconfiguration can be used to supplement automation. For example, the system may disconnect tube connections to a container, the user may replace the container and the system may connect the new container and start a new unit operation. The system may further check whether the user actions have been performed correctly. This example shows that the rather complex task of swapping out containers and placing them at the correct location does not need to be solved automatically with the present system while at the same time the correct replacement is checked by the user and the system.

Performing multiple unit operations within the same cartridge minimizes downtime by reducing the need for manual reconfiguration between processes, allowing for continuous processing of materials.

As specified in claim 7, the system may comprise mechanically defined positions, e.g. comprising small elevations, that allow precisely placing the cartridges and containers at the defined positions, for example by inserting the elevations into matching depressions of the respective cartridge or container. Mechanically defined positions make it easy for the user to place the cartridges and containers precisely and reproducibly such that the system can then localize the cartridges and containers. Proper alignment and handling of cartridges and containers minimize risks associated with incorrect connections or misalignment, which can lead to an automated action like welding failing.

In an embodiment according to claim 8, the system may support the user by visually indicating how the cartridge configuration should be reconfigured. Visual indications simplify the reconfiguration process for users by providing clear instructions on what steps to take and where to place components, reducing confusion and potential errors during the process. Clear visual cues help ensure that cartridges and containers are placed correctly and securely during the reconfiguration process, minimizing risks associated with incorrect placement or misalignment. Accurate reconfiguration instructions streamline the process, reducing downtime and improving overall system productivity.

The system may also comprise a storage location of containers, in particular an incubation location for incubation containers, and aid the user with storing and retrieving the correct containers (claim 9). Proper containment and storage of cell cultures minimize potential contamination risks and ensure that they remain in a controlled environment throughout the process. A dedicated storage location for containers allows for efficient management and retrieval of cell cultures during the process, ensuring that they are readily available when needed.

Claim 10 explains how in one embodiment the system may confirm the correct placement of a container at the correct location and then proceed further automatically after having confirmed the correct user action.

Claim 11 concerns a preferred boundary between system and user actions in the workflow and claim 12 specifies that preferably, a fluid transfer is done by the system between the cartridge and a container, thereby allowing replacement of the cartridge for the next user action.

Another teaching according to claim 13, which is of equal importance, relates to a bioprocessing system for performing a bioprocess on immune or naive cell cultures to obtain processed cell cultures on a semi-automated bioprocessing system, wherein the bioprocessing system comprises a primary workstation, wherein for performing the bioprocess the bioprocessing system performs a workflow, wherein the workflow comprises a plurality of unit operations performed by the primary workstation, wherein the primary workstation comprises a unit operation interface, wherein the unit operations are performed by an interaction of the unit operation interface with at least one cartridge, wherein the cartridge, optionally together with containers, placed at, in particular on, the unit operation interface define a cartridge configuration, wherein the unit operation interface comprises at least one active element interacting with the at least one cartridge to perform at least one unit operation of the plurality of unit operations, wherein a first unit operation and a second unit operation of the plurality of unit operations are performed by the unit operation interface with different cartridge configurations, wherein between the first and the second unit operation the cartridge configuration is manually reconfigured in a reconfiguration step, wherein the manual reconfiguration in the reconfiguration step is supported by the bioprocessing system.

All explanations given with regard to the proposed method are fully applicable. The bioprocessing system may be configured to perform any of the described method steps.

Another teaching according to claim 14, which is of equal importance, relates to a method for performing a further iteration of the bioprocess performed according to the proposed method as a previous iteration on an automated, in particular fully automated, further bioprocessing system, wherein the further bioprocessing system comprises a further primary workstation, wherein for performing the bioprocess the further bioprocessing system performs the workflow, wherein the plurality of unit operations are performed by the further primary workstation, wherein the further primary workstation comprises a further unit operation interface, wherein the unit operations are performed by an interaction of the further unit operation interface with at least one cartridge, wherein the cartridge, optionally together with containers, placed at, in particular on, the further unit operation interface define a cartridge configuration, wherein the further unit operation interface comprises at least one active element interacting with the at least one cartridge to perform at least one unit operation of the plurality of unit operations, wherein a first unit operation and a second unit operation of the plurality of unit operations are performed by the unit operation interface with different cartridge configurations, wherein between the first and the second unit operation the cartridge configuration is reconfigured in a reconfiguration step, wherein the reconfiguration in the reconfiguration step is performed at least partially, in particular fully, automatically.

As has been explained in the introduction, the proposed method and system may serve as a stepping stone on the way to a fully automated system. It is therefore proposed to increasingly automate the bioprocess with different systems up to a full automation. A main target of the increased automation is the reconfiguration which was done manually in the first proposed method. The further bioprocessing system may be substantially identical to the proposed bioprocessing system on a functional level. In a very preferred embodiment, the bioprocess is not changed with the change of the system and up to all system components directly influencing the bioprocess functionally stay the same.

Therefore, all explanations given with regard to the proposed method and the proposed bioprocessing system are fully applicable.

Claim 15 specifies preferred targets of the gradual automation.

In the following, embodiments of the invention are explained with respect to the drawing. The drawing shows in
- Fig. 1,: a proposed bioprocessing system,
- Fig. 2,: detail views of a) a container and b) a cartridge,
- Fig. 3,: tube welding with tube holders,
- Fig. 4,: schematically the change of cartridge configurations for two unit operations and
- Fig. 5,: an integrated bioprocessing system for performing the workflow fully automated.

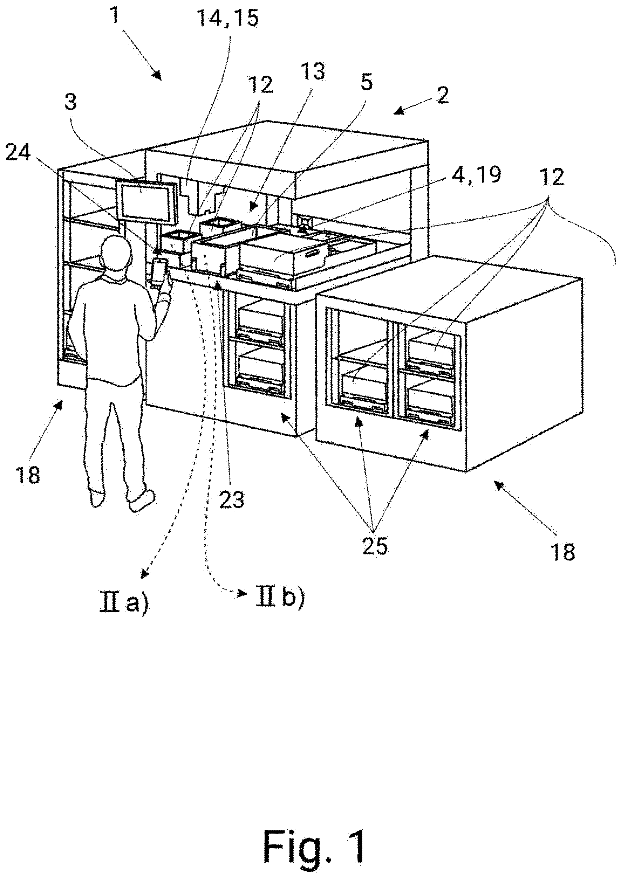

Proposed is a method for performing a bioprocess on immune or naive cell cultures to obtain processed cell cultures on a semi-automated bioprocessing system 1. Here and preferably, the processed cell cultures are destined for autologous or allogenic cell therapy. Fig. 1 shows an overview over an exemplary bioprocessing system 1. The bioprocessing system 1 comprises a primary workstation 2. The shown bioprocessing system 1 may be scalable and may therefore also comprise several primary workstations 2. The bioprocessing system 1 is used for performing a bioprocess on an individual cell culture. For example, the bioprocess may comprise unit operations such as "enrichment" and/or "selection" and/or "activation" and/or "loading" and/or "genetic modification" and/or "expansion" and/or "formulation and fill" and/or "wash" and/or "separation". A unit operation is, as generally used in the art, a change of the cell culture performed by one or more actions, preferably of the bioprocessing system 1.

For performing the bioprocess, the bioprocessing system 1 performs a workflow. Here and preferably the workflow is defined by a digital recipe which is executed by a control system of the bioprocessing system 1. Different steps of the workflow may be displayed on a user interface 3 of the bioprocessing system 1 for information of a user. The workflow comprises a plurality of unit operations performed by the primary workstation 2. Here it should be noted that the primary workstation 2 may also perform the same unit operation with different configurations.

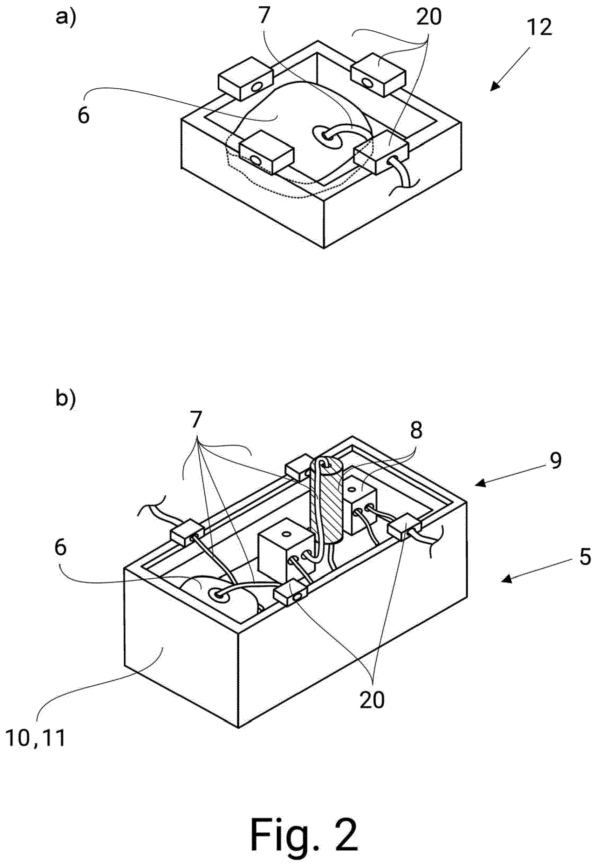

The primary workstation 2 comprises a unit operation interface 4. The unit operations are performed by an interaction of the unit operation interface 4 with at least one cartridge 5. A cartridge 5 preferably comprises at least one fluid vessel 6, in particular bag, and/or fluid conduits, in particular tubes 7 (Fig. 2b)). Additionally, a cartridge 5 preferably comprises at least one functional element 8 for interaction with the cell culture. The functional element 8 may be or form part of a pump, a centrifuge or a magnetic field generator for magnetic separation, for example. The cartridge 5, before being used, may comprise a preconfigured, in particular single-use, fluidic structure 9 and a, in particular multi-use, housing 10. The housing 10 preferably comprises a frame 11. The cartridge 5 may be preconfigured outside the bioprocessing system 1 by a user and then placed on the unit operation interface 4 such that a unit operation can be performed by a cooperation of the primary workstation 2 and the cartridge 5. For different unit operations, cartridges 5 with different fluidic structures 9 may be used. Fig. 2b) shows an exemplary cartridge 5.

The cartridge 5, optionally together with containers 12 (e.g. Fig. 2a)), placed at the unit operation interface 4 defines or define a cartridge configuration 13. When the term "container" is mentioned herein, it always refers to an additional container 12 and not the cartridge 5. Such a container 12 may comprise a fluid vessel 6 and/or fluid conduits. The cartridge 5 optionally with the containers 12 is here and preferably placed on the unit operation interface 4. As can be seen in Fig. 1, generally, the primary workstation 2 may be a workbench type workstation and therefore the cartridge 5 and the containers 12 may be placed horizontally next to each other. The cartridge configuration 13 comprises the configuration of the cartridge 5 itself and fluidic connections of the cartridge 5 to the containers 12. Therefore, two cartridges 5 with different fluidic structures 9 which comprise different functional elements 8, different tubes 7 or the like, have different cartridge configurations 13. Two cartridges 5 of the same type have the same cartridge configuration 13, which may be changed by different connections to different containers 12 though.

The unit operation interface 4 comprises at least one active element 14 interacting with the at least one cartridge 5 to perform at least one unit operation of the plurality of unit operations. The at least one active element 14 may comprise a yet to be explained weld robot 15. Additionally or alternatively, the at least one active element 14 may comprise an active element 14 powering a functional element 8 of the cartridge 5 and/or an active element 14 directly interacting with the cell culture, for example by pumping the cell culture. The at least one active element 14 may comprise additionally or alternatively a functional element 8 directly performing at least a part of the unit operation, for example a magnetic field generator. Ambient control, e.g. a general heating of the surroundings of the cartridge 5 is not considered an active element 14.

The unit operation interface 4 may comprise several active elements 14 for different unit operations. It should be noted that unrelated workbenches for different unit operations without overlapping usage for different unit operations are not considered to be one and the same unit operation interface 4. The unit operation interface 4 may additionally to the functional element 8 comprise connectors for signals, e.g. a bus connection or a wireless connection like Wi-Fi and/or an NFC reader and/or Bluetooth, and/or connectors for electrical energy. Here and preferably, cartridges 5 used for unit operations on the unit operation interface 4 are connected to at least one standard connector for electrical energy and/or signals in addition to above-mentioned active element 14 for at least one of the unit operations.

It is here the case that a first unit operation 16 and a second unit operation 17 of the plurality of unit operations are performed by the unit operation interface 4 with different cartridge configurations 13. Preferably, these different cartridge configurations 13 comprise different cartridges 5. Between the first and the second unit operation 16, 17 the cartridge configuration 13 is manually reconfigured in a reconfiguration step. The manual reconfiguration in the reconfiguration step is supported by the bioprocessing system 1. Here and preferably, the first and the second unit operation 16, 17, in particular the plurality of unit operations, are performed automatically without user intervention.

An exemplary use of the bioprocessing system 1 may start with placing a container 12 with the cell culture, possibly a container 12 with a medium, and a cartridge 5 for the first unit operation 16 on the unit operation interface 4, and then be followed by fluidically connecting the container 12 or containers 12 and the cartridge 5, transferring the cell culture and possibly medium into the cartridge 5, performing the first unit operation 16, transferring the cell culture back into the container 12, disconnecting the cartridge 5 and container 12 or containers 12, replacing, manually, the cartridge 5 with a second cartridge 5 for the second unit operation 17 and again transferring the cell culture and performing the second unit operation 17 in the second cartridge 5. Most of these steps can be performed automatically, however, the change of cartridges 5 would require a lot of supporting automation to move and place cartridges 5 which is not necessary for the workflow of the bioprocess itself. Leaving the change to the user simplifies the overall system and increases the flexibility of the system configuration. It becomes possible to automate some unit operations and scale the overall process by "adding" users for reconfiguration and transport steps under supervision of the bioprocessing system 1.

If the cartridges 5 have IDs readable by the bioprocessing system 1, as is preferred, the bioprocessing system 1 may check that the correct cartridge 5 has been placed at the unit operation interface 4, thereby also increasing the overall security as the correct workflow is then, at least partially, checked by the user and the bioprocessing system 1.

The bioprocessing system 1 may also comprise secondary workstations 18 dedicated to a single unit operation. Fig. 1 for example shows secondary workstations 18 for an incubation and/or expansion step. Other secondary workstations 18 like a controlled rate freezer are possible but not presently in focus. The bioprocessing system 1 may comprise more than one primary workstation 2 and/or more than one secondary workstation 18. To perform the bioprocess, some unit operations are performed at one primary workstation 2. Other unit operations may be performed at other primary workstations 2 and/or at a secondary workstation 18. Here and preferably, a transport between at least two primary workstations 2, in particular for consecutive unit operations, and/or between a primary workstation 2 and a secondary workstation 18, in particular for consecutive unit operations, the cell culture may be transported inside of a cartridge or container. In this way, the cell culture itself is not affected by transport operations. Therefore, here and preferably, at least two consecutive unit operations are performed on two different primary workstations 2 and/or a primary workstation 2 and a secondary workstation 18. The primary workstations 2 may be differently configured.

The bioprocessing system 1 shown in the drawings is preferably adapted to perform a bioprocess for the manufacturing of genetically modified T cells.

Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the cell culture is gradually processed. All explanations given are mainly directed to such a bioprocess. It may be pointed out, however, that those explanations are fully applicable to other bioprocesses as well.

The term "immune cell culture" is to be understood in a broad sense and refers to an immune cell culture comprising at least one type of immune cells. The immune cell culture may comprise other cell types that are not immune cells. Hence, the term "immune cell culture" refers to an immune cell culture at any stage during the bioprocess. Consequently, the type and fraction of immune cells present in the immune cell culture will change during the bioprocess applied as certain immune cells are enriched or depleted from the immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e.g. macrophages, and/or other cell types that are not immune cells, e.g., erythrocytes, and/or other subtypes of immune cells, e.g., CD8+ T cells, will be depleted from the immune cell culture. The immune cells to be enriched are referred to as target immune cells. Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

It should be noted that the bioprocessing system 1 is used for small scale par-allelized bioprocesses on individual cell cultures.

It may be the case that the same active element 14 interacts with the differently configured cartridges 5 to perform the first and the second unit operation 16, 17, and/or, that the active element 14 transfers mechanical energy to the cartridge 5 to perform the first and/or the second unit operation 16, 17. For the latter case, the active element 14 may preferably be a rotating shaft to drive a pump head or any other functional element 8 of the cartridge 5 that needs rotational mechanical energy.

During at least one of the plurality of unit operations here and preferably a container 12 is placed at, in particular on, the unit operation interface 4 and fluidically connected to the cartridge 5. The term "is placed at" means the state of being placed and connected, not the action of placing and connecting. Therefore, the bioprocessing system 1 performs at least one unit operation with a cartridge configuration 13 comprising a container 12. Preferably, the unit operation interface 4, in particular via the active element 14, pumps a liquid between the cartridge 5 and the container 12. For that, the cartridge 5 and/or the container 12 may comprise a pump head, and, the active element 14 may be a drive element for the pump head. Including the pump head in the cartridge 5 or container 12 makes it easier to correctly contact the pump head and the tubes 7 as this connection may be done by a user outside the bioprocessing system 1 when preconfiguring a fluidic structure 9 of the cartridge 5 or container 12. Here and preferably, during at least one unit operation performed at the unit operation interface 4, the cartridge configuration 13 comprises at least two containers 12, which may be different types of containers 12. The term "preconfigured" means that the fluidic structure 9 is inserted into the cartridge 5 or container 12 outside of the bioprocessing system 1.

Fig. 2a) shows an exemplary container 12 which comprises essentially only a bag for a medium or the cell culture. Fig. 1 shows in addition to two of those containers 12 on the left side of the cartridge 5 a different type of container 12 on the right side of the cartridge 5. The container 12 on the right side is an incubation container 12 which may be placed in the secondary workstations 18 together with the cell culture for incubation and/or expansion. The incubation container 12 may comprise functional elements 8 for incubation like sensors, elements for perfusion, an extendable bag and the like.

In a preferred embodiment, the primary workstation 2 comprises a welding interface 19 with a weld robot 15. The weld robot 15 may perform tube connections and/or seal tubes 7 and may additionally perform integrity testing on tube connections. The weld robot 15 here and preferably performs tube welding fluidically connecting and/or disconnecting the cartridge 5 to and/or from at least one container 12. Tube welding is a way of making sterile connections between tubes 7 with a high success rate and good reliability. Including automatic tube welding into the bioprocessing system 1 enables automatically connecting different cartridges 5 and containers 12 and transferring liquids as needed. The bioprocessing system 1 is therefore highly flexible. A detailed description of a preferred weld robot 15 may be found in patent application EP23162143.

One challenge is correctly identifying and interacting with tubes 7 at cartridges 5 and containers 12 that have been manually placed at the welding interface 19. Preferably, the weld robot 15 automatically grabs a tube 7 of the cartridge 5 and a tube 7 of the container 12 and automatically welds the tubes 7 together. For that, it is preferably the case that the cartridge 5 and/or container 12 and/or containers 12, in particular including the incubation containers 12, comprise each at least one tube holder 20 holding a tube 7 and that the weld robot 15 interacts with the tube holder 20 to perform the tube welding. Fig. 2 shows tube holders 20 on the container 12 in a) and the cartridge 5 in b). The tube holder 20 may be removably attached to the cartridge 5 and/or container 12. It is simpler to automate an interaction with a rigid tube holder 20 than with a non-rigid tube 7. The tube holders 20 may be able to extrude and/or retract the tube 7 they hold, in particular driven by the weld robot 15.

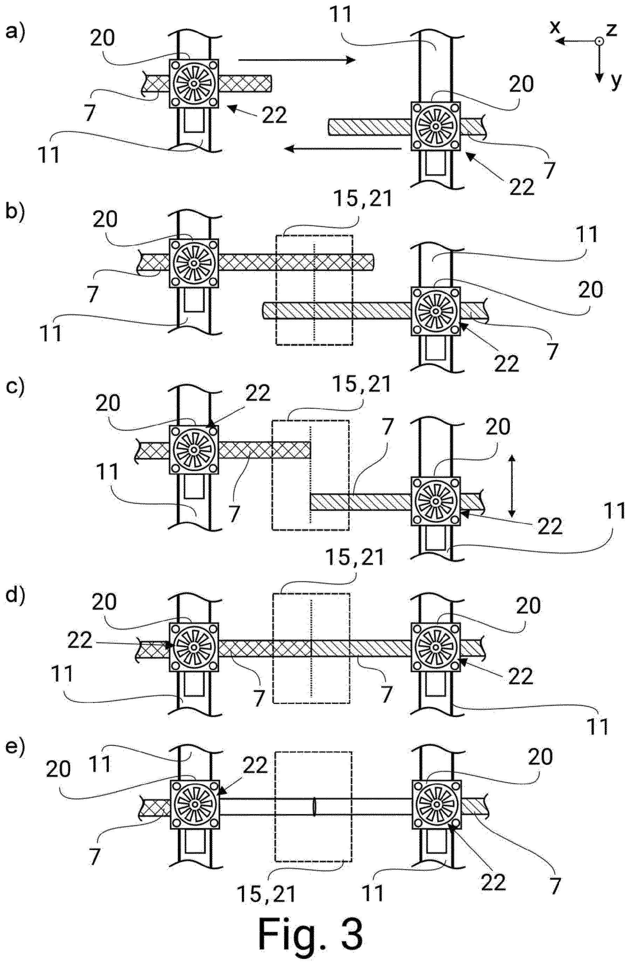

Fig. 3 shows how the tube welding may be performed. Here and preferably, the tube holders 20 are attached to a frame 11 of the cartridge 5 and/or container 12. The weld robot 15 may be adapted to dock onto the tube holders 20, thereby bringing the tube holders 20 and the weld robot 15 into well-defined positions with regard to each other. The tube holders 20 may be movable by the weld robot 15 or of their own accord along a frame 11 of the cartridge 5 and/or container 12. At the beginning of the tube welding, the tube holders 20 may be positioned with an offset to each other such that the tubes 7 are positioned next to each other. The tubes 7 may be extruded (indicated by the horizontal arrows in Fig. 3a)) and the tubes 7 and/or tube holders 20 may be grabbed by the weld robot 15 or a weld head 21 of the weld robot 15. Fig. 3 shows in a) the positioning with offset of the tube holders 20 and in b) the extruded tubes 7 schematically grabbed by the weld head 21. For example, the weld head 21 may be positioned relative to the tube holders 20, for example by docking onto the tube holders 20, and the weld head 21 may comprise one or two funnels into which the tubes 7 are extruded. The weld head 21 may cut (Fig. 3c)) and weld (Fig. 3d)) the tubes 7. Fig. 3e)) shows the connected state. Fig. 3 also shows that the tube holders 20 may comprise a drive mechanism 22 engageable by the weld robot 15 to extrude and/or retract the tubes 7.

It is here and preferably the case, that the unit operation interface 4 and the welding interface 19 are the same interface. The weld robot 15 is therefore usable as the active element 14 to perform tube connections and/or tube sealing. The weld robot 15 may be a three-axis, in particular three-linear-axis, robot. The welding interface 19 is then defined by the reach of this robot.

Alternatively, the unit operation interface 4 and the welding interface 19 are different and preferably distant from each other. It may then be the case, that the bioprocessing system 1 automatically transports the cartridge 5 and preferably the container 12 or containers 12 between the unit operation interface 4 and the welding interface 19, for example by means of a conveyor or another means of horizontal transfer. A simple conveyor between the interfaces is still easier to build and automate than a complex transport system for several cartridges 5 and containers 12.

In another embodiment, the bioprocessing system 1 does not automatically transport the cartridge 5 and the container 12 or containers 12 between the unit operation interface 4 and the welding interface 19, and, the cartridge 5 and preferably the container 12 or containers 12 are manually transported between the unit operation interface 4 and the welding interface 19.

A welding interface 19 distant from the unit operation interface 4 enables using one welding interface 19 for two or more unit operation interfaces 4, which consequently may be the case in one embodiment in which the bioprocessing system 1 comprises at least two unit operation interfaces 4. Alternatively or additionally, the welding interface 19 may be used for other welding operations not directly related to the performance of a unit operation by the unit operation interface 4.

According to one embodiment it is proposed, that the first and the second unit operations 16, 17 are performed with, in particular partially or completely within, the same cartridge 5, preferably after a manual reconfiguration of the cartridge 5 and/or after a connection of the cartridge 5 to at least one container 12. As has been explained above, the cartridge 5 may be exchanged between the first and second unit operation 16, 17. Alternatively, one or more containers 12 may be exchanged, or the cartridge 5 may be reconfigured in another way manually.

In another embodiment that simplifies the detection of and interaction with tubes 7, the unit operation interface 4 and/or the welding interface 19 comprise a, in particular mechanically, defined cartridge position 23 and preferably at least one, preferably at least two, in particular mechanically, defined container positions 24. The defined position or positions may comprise positioning elements, for example elevations, pins, or the like, that interact with corresponding counter positioning element, for example depressions, holes, or the like at the cartridge 5 and/or container 12. The positioning elements may be self-centering such that placing the cartridge 5 or container 12 on the elements is easy. Afterwards, the position of the cartridge 5 or container 12 is well-defined with regard to the bioprocessing system 1. Preferably, the weld robot 15 uses the defined cartridge position 23 and container position 24 or container positions 24 to detect the positions of the tubes 7.

Turning towards the user interaction, in the reconfiguration step the bioprocessing system 1 may visually indicate to a user how to perform the reconfiguration, in particular by indicating which cartridge 5 to reconfigure and/or which container 12 to reconfigure and/or which cartridge 5 to remove and/or where to place a cartridge 5 and/or which container 12 to remove and/or where to place a container 12. The visual indication may happen via the user interface 3 or locally at the unit operation interface 4, which may comprise light elements like LEDs. For example, the container 12 to be removed may have yellow LEDs lit around it or a position to place a container 12 at may be lit and turn green after correct placement of the container 12. Additionally or alternatively, for example, the position of an incubation container 12 at the secondary workstation 18 may be highlighted, for example lit if the incubation container 12 should be positioned at the unit operation interface 4.

In the reconfiguration step, the bioprocessing system 1 may detect a correct reconfiguration of the cartridge configuration 13, in particular by detecting the presence of the correct cartridge 5 and/or container 12 after reconfiguration at the correct position and/or the correct reconfiguration of the cartridge 5. The cartridge 5 and/or container 12 may comprise a digitally readable ID, for example stored in an NFC chip, as a barcode, or the like. The bioprocessing system 1 may digitally read the ID and compare it to the recipe for the workflow. The bioprocessing system 1 may comprise ID readers at the defined locations.

As has already been mentioned with regard to the secondary workstation 18, the bioprocessing system 1 here and preferably comprises a storage location 25 for containers 12, in particular incubation containers 12, containing the cell cultures. The storage locations 25 are here comprised by the secondary workstations 18. At the storage locations 25, the cell cultures in the incubation containers 12 can be incubated for a period of time, in particular during an expansion unit operation.

It is here and preferably the case, that the bioprocessing system 1 indicates to a user which container 12 to remove from the storage location 25 and place at the unit operation interface 4 and/or which container 12 to remove from the unit operation interface 4 and place at the storage location 25 and verifies the correct transfer of the container 12.

An exemplary use of the bioprocessing system 1 may comprise that for performing the first unit operation 16 the user places a container 12 from the storage location 25 at a defined container position 24 of the primary workstation 2, that the bioprocessing system 1 identifies the placement of the correct container 12 at the correct defined container position 24, that the user places at least one cartridge 5 at a defined cartridge position 23 of the primary workstation 2, that afterwards the weld robot 15 performs tube welding to fluidically connect the cartridge 5 and the container 12, that afterwards the primary workstation 2 performs the first unit operation 16 with the cartridge 5, and preferably, that afterwards the weld robot 15 fluidically disconnects the cartridge 5 and the container 12. Preferably, the weld robot 15 also performs an integrity test of the weld after connecting the tubes 7 and before transferring the liquid.

According to one embodiment it is proposed, that no user action is required while performing the unit operation and preferably while connecting and/or disconnecting the cartridge 5 and the container 12. Preferably, the bioprocessing system 1 detects a user action during the performance of the unit operation and preferably while connecting and/or disconnecting the cartridge 5 and the container 12. If the user action is detected during the performance of the unit operation, for example by machine vision, and preferably while connecting and/or disconnecting the cartridge 5 and the container 12, the bioprocessing system 1 may perform an abort procedure. The bioprocessing system 1 may also comprise a closable protecting element for protecting the process, for example a glass door. A closeable protecting element will also protect the user from the robotics. The bioprocessing system 1 would typically involve some degree of protection of the user from the robotics e.g. glass door, light curtain, motion detection or other interlock.

Further steps that may be performed include that the primary workstation 2 pumps the cell culture from the cartridge 5 used in the first unit operation 16 into a container 12 fluidically connected to the cartridge 5, that the weld robot 15 disconnects the cartridge 5 and the container 12, that the user replaces the cartridge 5 guided by the bioprocessing system 1 with a differently configured new cartridge 5, optionally that the bioprocessing system 1 verifies that the correct new cartridge 5 was placed at the correct position, that the weld robot 15 connects the new cartridge 5 to the container 12 containing the cell culture, that the primary workstation 2 pumps the cell culture from the container 12 into the new cartridge 5 and performs the second unit operation 17 with the second cartridge 5.

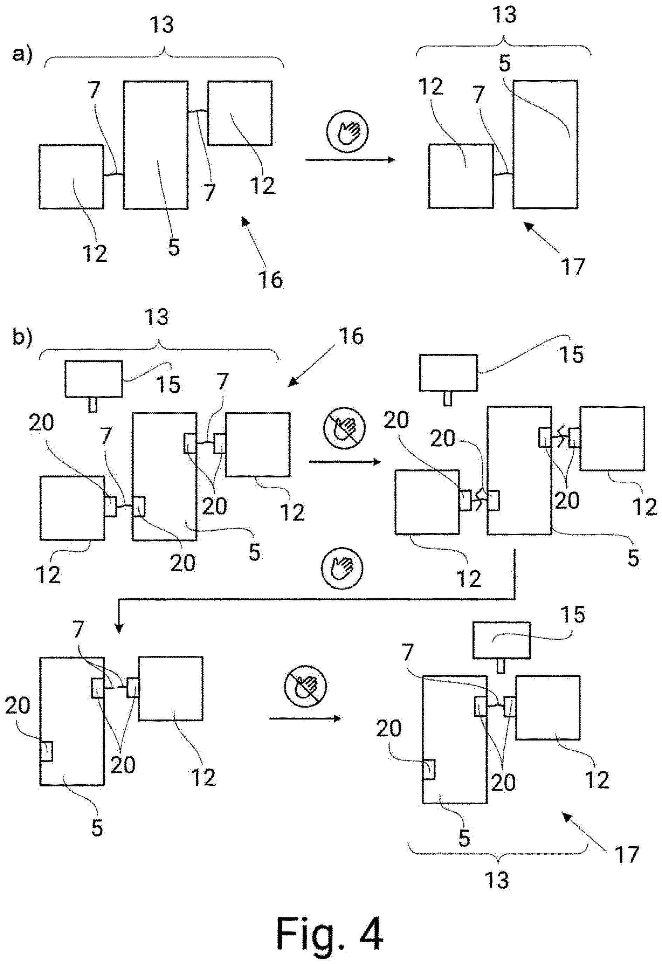

Fig. 4 shows schematically method steps for two exemplary unit operations. In a), the first unit operation 16 transitions with manual input to the second unit operation 17 with a different cartridge configuration 13. In b), this transition is shown more in detail with the weld robot 15 removing tube 7 connections in the first cartridge configuration 13 and connecting tubes 7 in the second cartridge configuration 13. In between, the user removes containers 12 from the first cartridge configuration 13 and places another container 12 at the unit operation interface 4 for the second cartridge configuration 13.

Turning towards the overall usage of the bioprocessing system 1, the method may comprise one or more of the following steps, in particular in this order:
- a vessel, in particular bag, with patient material arrives and comprises an ID, in particular barcode, for identification
- the vessel is placed in a, preferably rigid, container 12 that also has an ID, e.g. a barcode, and preferably human readable material
- the IDs are sent to the bioprocessing system 1, in particular by scanning both barcodes, to associate container 12 and vessel
- the bioprocessing system 1 plans what needs to be done and schedules tasks before or when the vessel arrives
- for the vessel or patient material a recipe has been defined that needs to be carried out, and the recipe also defines a rule set for that vessel, e.g. deviation handling
- certain media need to be prepared and a user is guided to prepare the correct amount and type of media for this recipe
- the bioprocessing system 1 forecasts what will be needed throughout the process and guides a user on preparation and storage of needed materials
- a user places materials in containers 12
- at some point a user is triggered by the bioprocessing system 1 to perform certain tasks, e.g., transfer of containers 12, docking and undocking from the unit operation interface 4.

It may be the case that in comparison to "usual" workflows, where a certain user is responsible for a certain vessel or patient material, here it may be the case that users respond to the bioprocessing system 1 and the bioprocessing system 1 schedules how the different processes on different vessels are performed and instructs the user to perform the steps as needed.

In one embodiment, the user negotiates actions to be performed by the user with the bioprocessing system 1. For example, the bioprocessing system 1 needs to negotiate with a user e.g. a cell count needed at 12 pm. The user may respond that it can be provided at 11 am. The bioprocessing system 1 may adapt a schedule based on the negotiation with the user.

The described example also serves to explain why, here and preferably, the cartridge 5 and/or container 12 comprises a multi-use shell, e.g. a tray, and a single-use insert, e.g. the fluid vessel 6. Here and preferably, the bioprocessing system 1 tracks the combination of multi-use shells and single-use inserts to verify manual actions performed.

Another teaching which is of equal importance relates to a bioprocessing system 1 for performing a bioprocess on immune or naive cell cultures to obtain processed cell cultures on a semi-automated bioprocessing system 1, wherein the bioprocessing system 1 comprises a primary workstation 2, wherein for performing the bioprocess the bioprocessing system 1 performs a workflow, wherein the workflow comprises a plurality of unit operations performed by the primary workstation 2, wherein the primary workstation 2 comprises a unit operation interface 4, wherein the unit operations are performed by an interaction of the unit operation interface 4 with at least one cartridge 5, wherein the cartridge 5, optionally together with containers 12, placed at, in particular on, the unit operation interface 4 define a cartridge configuration 13, wherein the unit operation interface 4 comprises at least one active element 14 interacting with the at least one cartridge 5 to perform at least one unit operation of the plurality of unit operations, wherein a first unit operation 16 and a second unit operation 17 of the plurality of unit operations are performed by the unit operation interface 4 with different cartridge configurations 13, wherein between the first and the second unit operation 16, 17 the cartridge configuration 13 is manually reconfigured in a reconfiguration step, wherein the manual reconfiguration in the reconfiguration step is supported by the bioprocessing system 1.

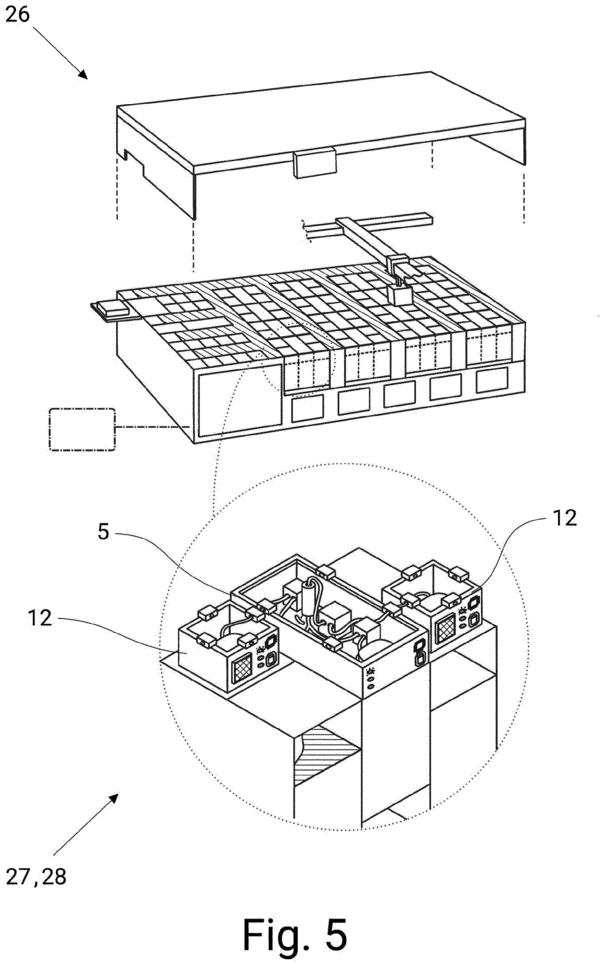

Another teaching which is of equal importance relates to a method for performing a further iteration of the bioprocess performed according to the proposed method as a previous iteration on an automated, in particular fully automated, further bioprocessing system 26. Fig. 5 shows an exemplary integrated bioprocessing system as a further bioprocessing system. The further bioprocessing system 26 comprises a further primary workstation 27, wherein for performing the bioprocess the further bioprocessing system 26 performs the workflow, wherein the plurality of unit operations are performed by the further primary workstation 27, wherein the further primary workstation 27 comprises a further unit operation interface 28, wherein the unit operations are performed by an interaction of the further unit operation interface 28 with at least one cartridge 5, wherein the cartridge 5, optionally together with containers 12, placed at, in particular on, the further unit operation interface 28 define a cartridge configuration 13, wherein the further unit operation interface 28 comprises at least one active element 14 interacting with the at least one cartridge 5 to perform at least one unit operation of the plurality of unit operations, wherein a first unit operation 16 and a second unit operation 17 of the plurality of unit operations are performed by the unit operation interface 4 with different cartridge configurations 13, wherein between the first and the second unit operation 16, 17 the cartridge configuration 13 is reconfigured in a reconfiguration step, wherein the reconfiguration in the reconfiguration step is performed at least partially, in particular fully, automatically.

It may be the case that gradually, further unit operations are performed by the bioprocessing system. At some point, the bioprocessing system may be swapped out with the further bioprocessing system, reducing the amount of user actions, preferably without losing any of the results of previous verifications of the automated steps.

According to one embodiment it is proposed, that some or all user actions necessary for the previous iteration are automated in the further iteration, and/or, that the reconfiguration is at least partially done by robotic transfer of the cartridge 5 and/or container 12.

## Claims

1. Method for performing a bioprocess on immune or naive cell cultures to obtain processed cell cultures on a semi-automated bioprocessing system (1), wherein the bioprocessing system (1) comprises a primary workstation (2), wherein for performing the bioprocess the bioprocessing system (1) performs a workflow, wherein the workflow comprises a plurality of unit operations performed by the primary workstation (2), wherein the primary workstation (2) comprises a unit operation interface (4),
wherein the unit operations are performed by an interaction of the unit operation interface (4) with at least one cartridge (5), wherein the cartridge (5), optionally together with containers (12), placed at, in particular on, the unit operation interface (4) define a cartridge configuration (13), wherein the unit operation interface (4) comprises at least one active element (14) interacting with the at least one cartridge (5) to perform at least one unit operation of the plurality of unit operations,
wherein a first unit operation (16) and a second unit operation (17) of the plurality of unit operations are performed by the unit operation interface (4) with different cartridge configurations (13), wherein between the first and the second unit operation (16, 17) the cartridge configuration (13) is manually reconfigured in a reconfiguration step, wherein the manual reconfiguration in the reconfiguration step is supported by the bioprocessing system (1).

2. Method according to claim 1, **characterized in that** the same active element (14) interacts with the differently configured cartridges (5) to perform the first and the second unit operation (16, 17), and/or, that the active element (14) transfers mechanical energy to the cartridge (5) to perform the first and/or the second unit operation (16, 17).

3. Method according to claim 1 or 2, **characterized in that** a container (12) is placed at, in particular on, the unit operation interface (4) and fluidically connected to the cartridge (5) during at least one of the plurality of unit operations, preferably, that the unit operation interface (4), in particular via the active element (14), pumps a liquid between the cartridge (5) and the container (12), more preferably, that the cartridge (5) and/or the container (12) comprises a pump head, and, that the active element (14) is a drive element for the pump head.

4. Method according to claim 3, **characterized in that** the primary workstation (2) comprises a welding interface (19) with a weld robot (15), that the weld robot (15) performs tube welding fluidically connecting and/or disconnecting the cartridge (5) to and/or from at least one container (12), preferably, that the weld robot (15) automatically grabs a tube (7) of the cartridge (5) and a tube (7) of the container (12) and automatically welds the tubes (7) together.

5. Method according to claim 4, **characterized in that** the unit operation interface (4) and the welding interface (19) are the same interface, or,
that the unit operation interface (4) and the welding interface (19) are different and preferably distant from each other, preferably, that the bioprocessing system (1) automatically transports the cartridge (5) and preferably the container (12) or containers (12) between the unit operation interface (4) and the welding interface (19), or, that the bioprocessing system (1) does not automatically transport the cartridge (5) and the container (12) or containers (12) between the unit operation interface (4) and the welding interface (19), and, that the cartridge (5) and preferably the container (12) or containers (12) are manually transported between the unit operation interface (4) and the welding interface (19).

6. Method according to one of the preceding claims, **characterized in that** the first and the second unit operations (16, 17) are performed with, in particular partially or completely within, the same cartridge (5), preferably after a manual reconfiguration of the cartridge (5) and/or after a connection of the cartridge (5) to at least one container (12).

7. Method according to one of the preceding claims, **characterized in that** the unit operation interface (4) and/or the welding interface (19) comprise a, in particular mechanically, defined cartridge position (23) and preferably at least one, preferably at least two, in particular mechanically, defined container positions (24), preferably, that the weld robot (15) uses the defined cartridge position (23) and container positions (24) to detect the positions of the tubes (7).

8. Method according to one of the preceding claims, **characterized in that** in the reconfiguration step the bioprocessing system (1) visually indicates to a user how to perform the reconfiguration, in particular by indicating which cartridge (5) to reconfigure and/or which container (12) to reconfigure and/or which cartridge (5) to remove and/or where to place a cartridge (5) and/or which container (12) to remove and/or where to place a container (12), and/or, that in the reconfiguration step the bioprocessing system (1) detects a correct reconfiguration of the cartridge configuration (13), in particular by detecting the presence of the correct cartridge (5) and/or container (12) after reconfiguration at the correct position and/or the correct reconfiguration of the cartridge (5).

9. Method according to one of the preceding claims, **characterized in that** the bioprocessing system (1) comprises a storage location (25) for containers (12), in particular incubation containers (12), containing the cell cultures, that the bioprocessing system (1) indicates to a user which container (12) to remove from the storage location (25) and place at the unit operation interface (4) and/or which container (12) to remove from the unit operation interface (4) and place at the storage location (25) and verifies the correct transfer of the container (12).

10. Method according to claim 9, **characterized in that** for performing the first unit operation (16) the user places a container (12) from the storage location (25) at a defined container position (24) of the primary workstation (2), that the bioprocessing system (1) identifies the placement of the correct container (12) at the correct defined container position (24), that the user places at least one cartridge (5) at a defined cartridge position (23) of the primary workstation (2), that afterwards the weld robot (15) performs tube welding to fluidically connect the cartridge (5) and the container (12), that afterwards the primary workstation (2) performs the first unit operation (16) with the cartridge (5), preferably, that afterwards the weld robot (15) fluidically disconnects the cartridge (5) and the container (12).

11. Method according to claim 10, **characterized in that** no user action is required while performing the unit operation and preferably while connecting and/or disconnecting the cartridge (5) and the container (12), preferably, that the bioprocessing system (1) detects a user action during the performance of the unit operation and preferably while connecting and/or disconnecting the cartridge (5) and the container (12), more preferably, that the bioprocessing system (1) performs an abort procedure if the user action is detected during the performance of the unit operation and preferably while connecting and/or disconnecting the cartridge (5) and the container (12).

12. Method according to one of claims 4 to 11, **characterized in that** the primary workstation (2) pumps the cell culture from the cartridge (5) used in the first unit operation (16) into a container (12) fluidically connected to the cartridge (5), that the weld robot (15) disconnects the cartridge (5) and the container (12), that the user replaces the cartridge (5) guided by the bioprocessing system (1) with a differently configured new cartridge (5), optionally that the bioprocessing system (1) verifies that the correct new cartridge (5) was placed at the correct position, that the weld robot (15) connects the new cartridge (5) to the container (12) containing the cell culture, that the primary workstation (2) pumps the cell culture from the container (12) into the new cartridge (5) and performs the second unit operation (17) with the second cartridge (5).

13. Bioprocessing system for performing a bioprocess on immune or naive cell cultures to obtain processed cell cultures on a semi-automated bioprocessing system (1), wherein the bioprocessing system (1) comprises a primary workstation (2), wherein for performing the bioprocess the bioprocessing system (1) performs a workflow, wherein the workflow comprises a plurality of unit operations performed by the primary workstation (2), wherein the primary workstation (2) comprises a unit operation interface (4),
wherein the unit operations are performed by an interaction of the unit operation interface (4) with at least one cartridge (5), wherein the cartridge (5), optionally together with containers (12), placed at, in particular on, the unit operation interface (4) define a cartridge configuration (13), wherein the unit operation interface (4) comprises at least one active element (14) interacting with the at least one cartridge (5) to perform at least one unit operation of the plurality of unit operations,
wherein a first unit operation (16) and a second unit operation (17) of the plurality of unit operations are performed by the unit operation interface (4) with different cartridge configurations (13), wherein between the first and the second unit operation (16, 17) the cartridge configuration (13) is manually reconfigured in a reconfiguration step, wherein the manual reconfiguration in the reconfiguration step is supported by the bioprocessing system (1).

14. Method for performing a further iteration of the bioprocess performed according to the method of one of claims 1 to 12 as a previous iteration on an automated, in particular fully automated, further bioprocessing system (26),
wherein the further bioprocessing system (26) comprises a further primary workstation (27), wherein for performing the bioprocess the further bioprocessing system (26) performs the workflow, wherein the plurality of unit operations are performed by the further primary workstation (27), wherein the further primary workstation (27) comprises a further unit operation interface (28), wherein the unit operations are performed by an interaction of the further unit operation interface (28) with at least one cartridge (5), wherein the cartridge (5), optionally together with containers (12), placed at, in particular on, the further unit operation interface (28) define a cartridge configuration (13), wherein the further unit operation interface (28) comprises at least one active element (14) interacting with the at least one cartridge (5) to perform at least one unit operation of the plurality of unit operations,
wherein a first unit operation (16) and a second unit operation (17) of the plurality of unit operations are performed by the unit operation interface (4) with different cartridge configurations (13), wherein between the first and the second unit operation (16, 17) the cartridge configuration (13) is reconfigured in a reconfiguration step, wherein the reconfiguration in the reconfiguration step is performed at least partially, in particular fully, automatically.

15. Method according to claim 14, **characterized in that** some or all user actions necessary for the previous iteration are automated in the further iteration, and/or, that the reconfiguration is at least partially done by robotic transfer of the cartridge (5) and/or container (12).
